# EUROPEAN PATENT SPECIFICATION

(11) **EP 0 623 137 B1**
(45) Date of publication and mention of the grant of the patent: **10.03.1999**
(21) Application number: 93902162.2
(22) Date of filing: 11.01.1993
(51) Int. Cl.: C07H 17/08, A01N 43/90, A61K 31/70, A23K 1/17

(54) **ANTIPARASITIC AGENTS**
ANTIPARASITISCHES MITTEL
AGENTS ANTIPARASITAIRES

(30) Priority: 24.01.1992 GB 9201505
(43) Date of publication of application: 09.11.1994
(73) Proprietor: Pfizer Limited, Sandwich Kent CT13 9NJ (GB); PFIZER INC., New York, N.Y. 10017 (US)
(72) Inventor: BANKS, Bernard Joseph, Sandwich, Kent CT13 9NJ (GB)
(74) Representative: Moore, James William, Dr.
(86) International application number: EP9300036
(87) International publication number: WO9315099

(56) References cited:
- EP-A- 0 008 184
- EP-A- 0 214 731
- EP-A- 0 238 258
- EP-A- 0 307 224

## Description

This invention relates to antiparasitic agents and in particular to compounds for use with domestic companion animals. The compounds are related to the avermectins but have modified substituent groups at the C-23 and C-25 positions. Processes for preparation of the compounds and compositions thereof are also included.

The avermectins and milbemycins form an important group of broad spectrum antiparasitic agents possessing anthelmintic, ectoparasiticidal, insecticidal and antibacterial activity, with application in the areas of animal and human health, agriculture and horticulture. The avermectins are a group of macrolide compounds (previously referred to as C-076 compounds) isolated from the fermentation broth of an avermectin producing strain of Streptomyces avermitilis. In addition to these fermentation derived products, a large number of publications describe compounds derived semisynthetically from these products, many of which possess useful antiparasitic activities. Some of this chemistry is reviewed in Macrolide Antibiotics, Omura S., Ed., Academic press, New York (1984) and by Davies, H.G., Green, R.H. in Natural Product Reports, (1986), 3, 87-121 and in Chem. Soc. Rev., (1991), 20, 211-269 and 271-239. Thus for example U.S. patent no 4200581 discloses avermectin derivatives substituted by hydrocarbon groups.

In our European Patent Application nos. 0214731 and 0317148, we describe the preparation of compounds related to the avermectins but having an unnatural substituent group at the C-25 position in place of the isopropyl or sec-butyl group which is present in the naturally occurring avermectins.

The present invention provides a series of semi-synthetically derived novel compounds wherein both the C-23 and C-25 position substituents are modified. These compounds form the starting point for a further series of semi-synthetically derived analogues wherein the C-5 and C-13 position substituents may also be modified. The compounds possess a broad spectrum of activity against insect pests, acari, free-living nematodes and parasites affecting animals. Moreover the compounds of the invention possess a number of beneficial properties compared to similar compounds in terms of their efficacy, pharmacokinetics and toleration. The benefits that arise from this unexpected combination of properties include efficacy against the important parasitic worms or arthropods afflicting livestock, domesticated animals or humans at lower doses than are currently employed for related compounds and, in addition, the ability to treat animals previously regarded as sensitive to this class of macrolide with a greater margin of safety.

Thus according to the present invention there are provided compounds having the formula:- 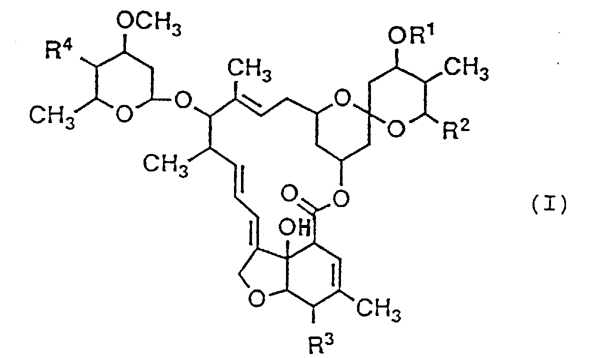 wherein
R¹ is C₁-C₆ alkyl;
R² is C₃-C₈ cycloalkyl ;
R³ is OH, C₁-C₄ alkoxy or R³ is linked by a double bond and is =N-OH; and
R⁴ is HO or a group of the formula:

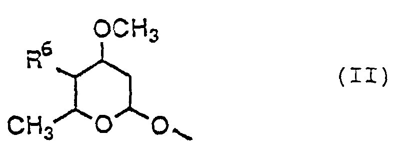

In the above definitions alkyl groups containing 3 or more carbon atoms may be straight or branch-chain; halo means fluoro, chloro, bromo, or iodo; and aryl means phenyl optionally substituted by one or more C₁-C₄ alkyl or C₁-C₄ alkoxy groups or halo atoms.

The C-076 complex comprises eight distinct but closely related compounds described as C-076 A1a, A1b, A2a, A2b, B1a, B1b, B2a and B2b. The "a" series of compounds refers to the natural avermectins wherein the 25-substituent is (5)-sec-butyl and the "b" series to those wherein the 25-substituent is isopropyl. The designations "A" and "B" refer to avermectins wherein the 5-substituent is methoxy or hydroxy, respectively, and the numeral "1" refers to avermectins wherein a double bond is present at the 22-23 position, and numeral "2" to avermectins lacking the 22-23 double bond and having a hydrogen at the 22-position and hydroxy at the 23 position.

In this specification, the "a" and "b" identifiers have been dropped, however, identifiers A1, A2, B1 and B2 have been retained to refer to non-natural avermectins having the structural features corresponding to those of the natural avermectins as noted above.

Compounds of the formula (I) wherein R³ is HO (avermectin B derivatives) are generally preferred. R¹ is preferably C₁-C₄ alkyl especially methyl or ethyl; R² is preferably cyclohexyl. Also preferred are compounds where R³ is =N-OH(oximino).

The compounds of formula (I) wherein R⁴ is α-L-oleandrosyl and R³ is OH or OCH₃ are prepared from the corresponding C-25 modified avermectin A2 or B2 derivative of formula (I) wherein R¹ is H, by reacting with a halide of the formula R¹-hal wherein hal is bromine or preferably iodine, in the presence of a silver salt.

The reaction is performed by stirring the appropriate avermectin having a hydroxy group at the C-23 position, with the halide in an organic solvent, in the presence of a suitable silver salt, preferably silver salicylate. We have found that diethyl ether is a preferred solvent. A period of several days at room temperature may be required for the reaction to go substantially to completion. Under these conditions we have surprisingly found that the reaction is substantially selective for the C-23 hydroxy group and it is not necessary to protect the C-5 hydroxy group present in the avermectin B class of compounds. The iodide is generally the preferred halide, however in activated compounds e.g. where R¹ is allyl, or benzyl, the bromide is preferable. The product is isolated after filtration and evaporation of the solvent and is purified if necessary, by chromatography.
Compounds of formula (I) wherein R³ is =NOH are prepared from the corresponding compound wherein R³ is hydroxy by oxidation, for example using manganese dioxide, to give the 5-oxo intermediate, which is then reacted with hydroxylamine to yield the oxime derivative (R³=NOH).

Compounds of the formula (I) wherein R⁴ is OH (monosaccharide derivatives) are prepared by selective hydrolysis of the appropriate avermectin starting material where R⁴ is α-L-oleandrosyl.

Appropriate reagents and conditions for the these steps may be determined by reference to literature precedents and to the experimental examples included hereafter.

The starting materials of formula (I) wherein R¹ is H are obtained directly from fermentation as previously described in EP-B-0214731 or EP-A-0317148.

As previously mentioned the compounds of the invention are highly active antiparasitic agents. Thus the compounds are effective in treating a variety of conditions caused by endoparasites including, in particular, helminthiasis which is most frequently caused by a group of parasitic worms described as nematodes and which can cause severe economic losses in swine, sheep, horses and cattle as well as affecting domestic animals and poultry. The compounds are also effective against other nematodes which affect various species of animals including, for example, Dirofilaria in dogs and various parasites which can infect animals and humans including gastro-intestinal parasites such as Ancylostoma, Necator, Ascaris, Strongyloides, Trichinella, Toxocara, canillaria, Trichuris, Enterobius and parasites which are found in the blood or other tissues and organs such as filiarial worms and the extra intestinal stages of Strongyloides, Trichinella and Toxocara.

The compounds are also of value in treating ectoparasite infections including in particular arthropod ectoparasites such as ticks, mites, lice, fleas, blowfly and biting insects.

The compounds of formula (I) are administered as a formulation appropriate to the specific use envisaged and to the particular species of host animal being treated and the parasite or insect involved. They may be administered by injection, either subcutaneously or intramuscularly. Alternatively they may be administered orally in the form of a capsule, bolus, tablet, chewable tablet or liquid drench, or they may be administered as a pour-on or spot-on formulation or as an implant. Such formulations are prepared in a conventional manner in accordance with standard veterinary practice. Thus injectable formulations may be prepared in the form of a sterile solution or emulsion. Capsules, boluses or tablets may be prepared by mixing the active ingredient with a suitable finely divided diluent or carrier, additionally containing a disintigrating agent and/or binder such as starch, lactose, talc, or magnesium stereate. A drench formulation may be prepared by dispersing the active ingredient in an aqueous solution together with dispersing or wetting agents. Pour-on or spot-on formulations may be prepared by dissolving the active ingredient in an acceptable liquid carrier vehicle, such as butyl digol, liquid paraffin or non-volatile ester with or without addition of a volatile component such as isopropanol. These formulations will vary with regard to the weight of active compound depending on the species of host animal to be treated, the severity and type of infection and the body weight of the host. Generally for oral or parenteral administration, a dose of from about 0.001 to 10 mg per kg, preferably 0.01 to 1 mg/kg of animal body weight given as a single dose or in divided doses for a period of from 1 to 5 days will be satisfactory but of course there can be instances where higher or lower dosage ranges are indicated and such are within the scope of this invention.

As an alternative the compounds may be administered with the animal feedstuff and for this purpose a concentrated feed additive or premix may be prepared for mixing with the normal animal feed.

For use as an insecticide the compounds are applied as sprays, dusts, emulsions, pour-on, spot-on formulations and the like in accordance with standard veterinary practice.

The invention is illustrated by the following Examples: Fast atom bombardment (FAB) mass spectrometry was performed on a VG model 7070E mass spectrometer using a sample matrix of glycerol, thiglycerol, water and sodium chloride. Electron impact (EI) mass spectrometry as performed using a VG model 7070F mass spectrometer. m/z values are quoted for the principal fragments. ¹H Nuclear magnetic resonance (NMR) spectral data were obtained on a Nicolet QE 300 spectrometer with a sample concentration of 5 mg/ml in deuterochloroform. The chemical shifts are given in parts per million relative to tetramethylsilane.

### EXAMPLE 1

### 23-Methoxy-22,23-dihydro-25-cyclohexylavermectin B1

A solution of 25-cyclohexylavermectin B2 (50 mg) and methyl iodide (570 mg) in diethyl ether (10 ml) containing a suspension of silver salicylate (200 mg) was stirred at room temperature for 30 hours. The reaction mixture was filtered and the filtrate evaporated to yield a yellow oil. The oil was purified by reverse phase high performance liquid chromatography on a Dupont Zorbax (trade mark) ODS C18 column eluting with a 15:85 mixture of water:methanol. Evaporation of the appropriate fractions gave the product (45 mg) as a white powder.
- FAB mass spectrometry:: (M+Na⁺) observed at m/z 930 (theoretical 930)
- EI mass spectrometry:: 623, 440, 363, 331, 247, 219, 195, 179, 167, 145, 135, 113, 95, 87.
¹H NMR as expected for a 22,23-dihydro avermectin B1 with a characteristic peak for the C-23 substituent at δ3.33 (3H,s,-OCH₃).

### EXAMPLES 2-5

The following Examples were prepared following the method of Example 1 from 25-cyclohexylavermectin B2 using the appropriate iodide

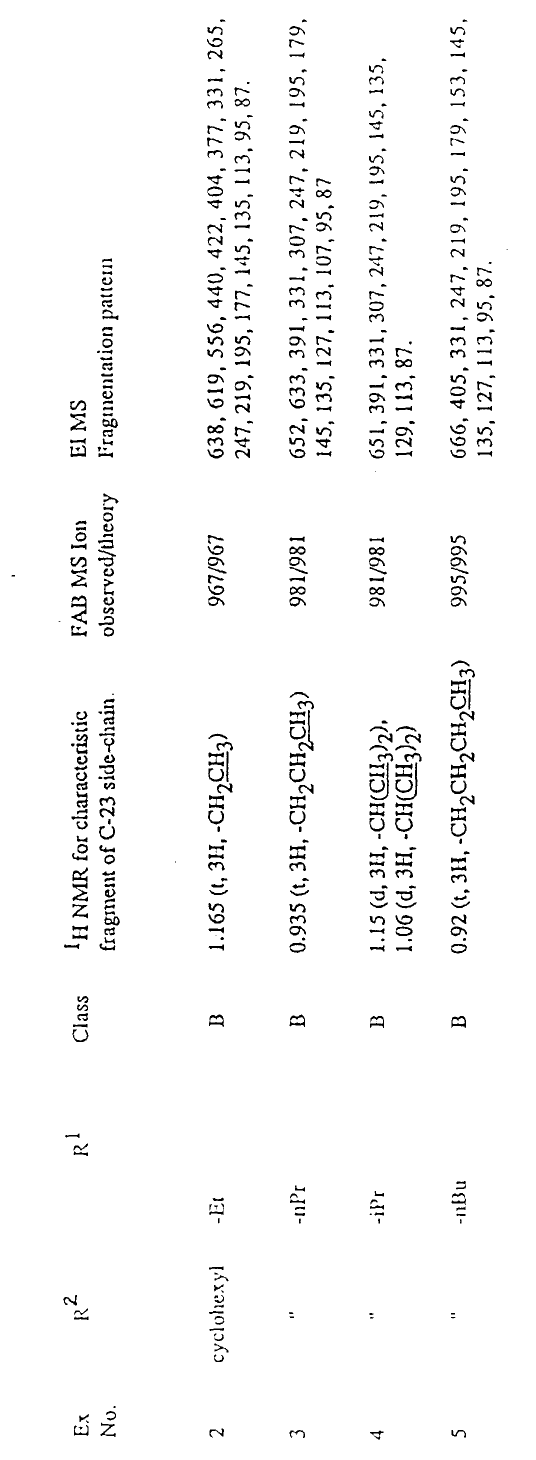

### EXAMPLE 6

### 23-Methoxy-5-oximino-22,23-dihydro-25-cyclohexylavermectin B1

A mixture of 23-methoxy-22,23-dihydro-25-cyclohexylavermectin B1 (1 g) and manganese dioxide (2 g) in dry diethyl ether (30 ml) was stirred at room temperature for 16 hours. Further manganese dioxide (1 g) was then added and stirring continued for a further 5 hours. The mixture was then filtered and the residue washed with dichloromethane (50 ml). The filtrate was evaporated to give 23-methoxy-5-oxo-22,23-dihydro-25-cyclohexylavermectin B1 (1 g) as a yellow foam. This was dissolved in pyridine (10 ml) and hydroxylamine hydrochloride (1 g) was added. The mixture was stirred at room temperature for 3 hours and then concentrated under vacuum to a small volume (3 ml) and partitioned between dichloromethane and 20% aqueous citric acid. The organic layer was separated, washed with 20% citric acid, then water, dried over anhydrous sodium sulphate, filtered and the solvent evaporated to give crude product (1.018 g). The product was purified by column chromatography on silica gel (100 g) eluting with dichloromethane/ethyl acetate (2:1) to give 23-methoxy-5-oximino-22,23-dihydro-25-cyclohexylavermectin B1 (711 mg). The product was further purified by reverse phase high performance liquid chromatography on a Dynamax (trade mark) 5 cm diameter ODS C18 column eluting with a mixture of methanol:water (85:15). Evaporation of appropriate fractions gave the pure title product (298 mg).
- FAB mass spectrometry:: (M+Na⁺) observed at m/z 966 (theoretical 966)
- EI mass spectrometry:: 637, 482, 363, 331, 289, 279, 274, 257, 251, 247, 219, 195, 179, 145, 127, 113, 111, 95, 87.
- Selected ¹H NMR data (δ):: 1.93 (3H,s) ; 3.3 (3H,s) ; 3.39 (3H,s); 3.4(3H,s); 8.62(1H,bs).

### EXAMPLE 7

### 23-Ethoxy-5-oximino-22 23-dihydro-25-cyclohexylavermectin B1

To a solution of 23-ethoxy-5-oxo-22,23-dihydro-25-cyclohexylavermectin B1 (0.8 g, see Preparation 3) in methanol (16 ml) and dioxan (16 ml) was added a solution of hydroxylamine hydrochloride (1 g) in water (16 ml). The mixture was warmed to 50°C and maintained at this temperature for 1 hour. The cooled solution was then evaporated and the residue partitioned between diethyl ether (100 ml) and water (100 ml). The organic layer was separated, washed with aqueous sodium hydrogen carbonate (100 ml, 5% solution) and water (100 ml), dried (MgSO₄) and evaporated. The crude product (0.8 g) was purified by column chromatography on silica gel (40 g) eluting with dichloromethane:ethyl acetate (100:0 to 70:30). Combination of appropriate fractions gave 23-ethoxy-5-oximino-22,23-dihydro-25-cyclohexylavermectin B1 (0.5 g). The product was further purified by reverse phase high performance liquid chromatography on a Dynamax (trade mark) 5 cm diameter ODS C18 column eluting with a mixture of methanol:water (90:10). Evaporation of appropriate fractions gave the pure title compound (380 mg).
- FAB mass spectrometry:: (M+Na⁺) observed at m/z 980 (theorectical 980)
- EI mass spectrometry:: 377, 331, 293, 289, 274, 265, 257, 247, 219, 195, 179, 145, 127, 113, 111, 95, 87.
- Selected ¹H NMR data (δ) :: 8.31 (bs,1H)

### EXAMPLE 8

### 23-Methoxy-22,23-dihydro-25-cyclohexylavermectin B1 monosaccharide

23-Methoxy-22,23-dihydro-25-cycolohexylavermectin B1 (10 g) was added to a 1% solution of sulphuric acid in isopropanol (100 ml). The mixture was stirred at room temperature for 16 hours. The reaction mixture was poured onto ice (100 g) and water (100 ml) and extracted with dichloromethane (2 x 100 ml). The combined organic extracts were washed with aqueous potassium hydrogen carbonate (50 ml, 20% solution) and water (25 ml), dried (NaSO₄) and evaporated to give an off-white solid. This was purified by column chromatography on silica gel (100 g) eluting with dichloromethane:ethyl acetate (2:1 to 1:1). Evaporation of appropriate fractions gave 8.8 g of a white solid 2 g of this solid was further purified by reverse phase high performance liquid chromatography on a Dynamax (trade mark) 5 cm diameter ODS C-18 column eluting with methanol:water (85:15). Evaporation of appropriate fractions gave 23-methoxy-22,23-dihydro-25-cyclohexyl-avermectin B1 monosaccharide (1.6 g) as a white amorphous powder.
- FAB mass spectrometry:: (M+Na⁺) observed at 809 (theoretical 809)
- EI mass spectrometry:: 624, 482, 363, 331, 279, 261, 251, 247, 227, 195, 179, 145, 143, 127, 113, 111, 95, 87.
- Selected ¹H NMR data (δ) :: 3.46 (s,3H), 3.3 (s,3H).

### EXAMPLE 9

### 23-Methoxy-5-oximino-22,23-dihydro-25-cyclohexylavermectin B1 monosaccharide

A mixture of 23-methoxy-22,23-dihydro-25-cyclohexyl-avermectin B1 monosaccharide (1.2 g) and manganese dioxide (5 g) in anhydrous diethyl ether (30 ml) was stirred for 2 hours. Further manganese dioxide (1 g) was added and stirring continued for 1 hour. The reaction mixture was filtered and evaporated. The residue (1 g) was taken up in methanol (20 ml) and dioxan (20 ml) and a solution of hydroxylamine hydrochloride (1 g) in water (20 ml) was added. The mixture was heated to 50°C for 1 hour, then cooled and evaporated. The residue was partitioned between diethyl ether (100 ml) and water (100 ml). The organic layer was separated, washed with aqueous sodium hydrogen carbonate (100 ml, 10% solution) and water (100 ml), dried (Na₂SO₄) and evaporated to give an oil (1 g) which was purified by column chromatography on silica gel (50 g) eluting with dichloromethane:ethyl acetate (100:0 to 75:25). Evaporation of appropriate fractions gave a foam which was further purified by reverse phase high performance liquid chromatography on a Dynamax (trade mark) 5 cm diameter ODS C-18 column eluting with methanol:water (85:15). Evaporation of appropriate fraction gave 23-methoxy-5-oximino-22,23-dihydro-25-cyclohexylavermectin B1 monosaccharide (160 mg) as a white amorphous powder.
- FAB mass spectrometry:: (M+Na⁺) observed at m/z 822 (theoretical 822)
- EI mass spectrometry:: 799, 655, 637, 482, 363, 331, 288, 279, 256, 251, 237, 219, 195, 179, 145, 127, 113, 111, 95, 87.
- Selected ¹H NMR data (δ):: 8.55 (bs,1H)

### EXAMPLE 10

### 23-Ethoxy-5-oximino-22,23-dihydro-25-cyclohexylavermectin B1 monosaccharide

To a solution of 23-ethoxy-22,23-dihydro-25-cyclohexyl-avermectin B1 (3.5 g) in isopropanol (35 ml) was added isopropanol (35 ml) containing sulphuric acid (0.7 ml). The mixture was allowed to stand for 16 hours at room temperature, then poured onto ice (175 g) and water 175 ml) and extracted with dichloromethane (200 ml, x2). The combined organic extracts were dried (MgSO₄) and evaporated to give a yellow foam (4.2 g); 2 g of which was dissolved in anhydrous diethyl ether (55 ml). To this stirred solution at room temperature was added manganese dioxide (10 g) and stirring continued for 2 hours after which manganese dioxide (1 g) was added and stirring continued for a further ½ hour. The reaction mixture was then filtered and evaporated to give a yellow foam (1.2 g) which was dissolved in methanol (24 ml) and dioxan (24 ml). A solution of hydroxylamine hydrochloride (1.2 g) in water (12 ml) was added. The mixture was stirred for 1 hour at 50°C then cooled and evaporated. The residue was partitioned between diethyl ether (100 ml) and water (100 ml). The organic layer was washed with aqueous sodium hydrogen carbonate (100 ml, 10% solution) and water (100 ml), dried (MgSO₄) and evaporated. The product was purified by column chromatography on silica gel (50 g) eluting with dichloromethane:ethyl acetate (100:0 to 80:20). Evaporation of appropriate fractions gave a foam (610 mg). This material was further purified by reverse phase high performance liquid chromatography on a Dynamax (trade mark) 5 cm diameter ODS C-18 column eluting with methanol:water (85:15). Evaporation of appropriate fractions gave 23-ethoxy-5-oximino-22,23-dihydro-25-cyclohexyl-avermectin B1 monosaccharide (340 mg) as a white amorphous powder.
- FAB mass spectrometry:: (M+Na⁺) observed at m/z 836 (theoretical 836)
- EI mass spectrometry:: 669, 651, 377, 331, 293, 274, 265, 247, 241, 219, 195, 179, 157, 145, 127, 113, 111, 95, 87.
- Selected ¹H NMR data (δ):: 8.48 (bs,1H)

### EXAMPLE 11

### 23-Methoxy-22,23-dihydro-25-cyclohexylavermectin A1

A solution of 23-methoxy-22,23-dihydro-25-cyclohexylavermectin B1 (250 mg) and methyl iodide (1 ml) in diethyl ethyl (10 ml) containing a suspension of silver oxide (250 mg) was stirred at room temperature for 48 hours. The reaction mixture was filtered and the filtrate evaporated to yield an oil which was purified by reverse phase high performance liquid chromatography on a Dynamax (trade mark) 5 cm diameter ODS C18 column eluting with a mixture of methanol and water (87:13). Evaporation of appropriate fractions gave pure title compound (139 mgs) as a white amorphous powder.
- FAB mass spectrometry:: (M+ Na⁺) observed at ^{m}/z 967 (theoretical 967)
- EI mass spectrometry:: 638, 482, 363, 331, 279, 275, 257, 251, 247, 219, 195, 193, 145, 127, 113, 111, 95, 87.
- Selected ¹H-NMR data (δ) :: 3.51(s,3H), 3.41(s,3H), 3.39(s,3H), 3.30(s,3H).

### PREPARATION 1

### 23-Ethoxy-5-oxo-22,23-dihydro-25-cyclohexylavermectin B1

A mixture of 23-ethoxy-22,23-dihydro-25-cyclohexylavermectin B1 (2 g) and manganese dioxide (4 g) in anhydrous diethyl ether (60 ml) was stirred at room temperature for 16 hours, further manganese dioxide (1 g) was then added and stirring continued for 48 hours. The mixture was then filtered and evaporated to give 23-ethoxy-5-oxo-22,23-dihydro-25-cyclohexylavermectin B1 as a yellow solid (1.6 g) which was used without purification.

## Claims

1. A compound having the formula: 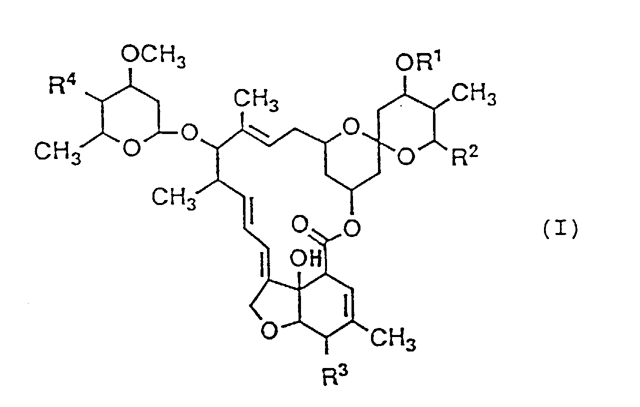 wherein
R¹ is C₁-C₆ alkyl;
R² is C₃-C₈ cycloalkyl;
R³ is OH, C₁-C₄ alkoxy or R³ is linked by a double bond and is =N-OH; and
R⁴ is HO, or a group of the formula

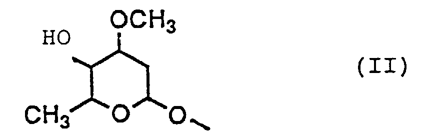

2. A compound as claimed in claim 1 wherein R³ is OH.

3. A compound as claimed in claim 1 wherein R³ is = N-OH.

4. A compound as claimed in claim 1 wherein R¹ is methyl or ethyl.

5. A compound as claimed in any one of claims 1 to4 wherein R² is cyclohexyl.

6. A composition for the treatment and prevention of parasitic infections in humans and animals, including ectoparasiticidal, insecticidal, acaricidal and anthelmintic compositions, which comprises a compound of the formula (I) as claimed in any one of claims 1 to 5 together with an inert diluent or carrier.

7. A composition as claimed in claim 6 in the form of a liquid drench or an oral, pour-on spot on formulation or in the form of an animal feedstuff or a premix or supplement for addition to animal feed.

8. A method of combating agricultural or horticultural pest infestations, which comprises applying an effective amount of a compound of the formula (I) as claimed in any one of the claims 1 to 5 to the organism responsible for said infection or infestation or to the location thereof.

9. The use of a compound of claim 1 for the preparation of a composition for treating insect or parasite infections or infestations in humans or animals.

## Patentansprüche

1. Verbindung der Formel: 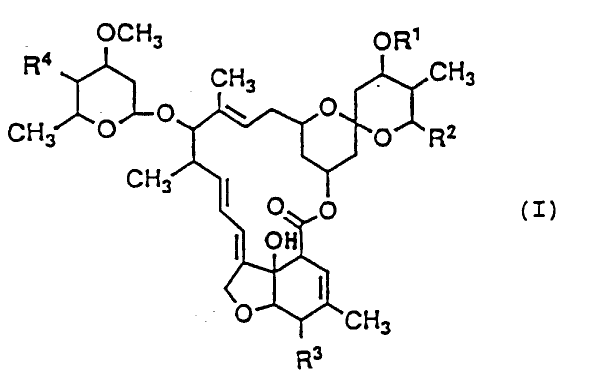 worin
R¹ C₁-C₆-Alkyl darstellt;
R² C₃-C₈-Cycloalkyl darstellt;
R³ OH, C₁-C₄-Alkoxy darstellt oder R³ durch eine Doppelbindung verbunden ist und =N-OH darstellt; und
R⁴ HO oder eine Gruppe der Formel 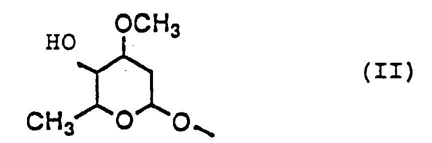 darstellt.

2. Verbindung nach Anspruch 1, worin R³ OH darstellt.

3. Verbindung nach Anspruch 1, worin R³ =N-OH darstellt.

4. Verbindung nach Anspruch 1, worin R¹ methyl oder Ethyl darstellt.

5. Verbindung nach einem der Ansprüche 1 bis 4, worin R² Cyclohexyl darstellt.

6. Zusammensetzung zur Behandlung und Verhinderung von parasitären Infektionen bei Menschen und Tieren, einschließlich ectoparasitizide, insektizide, akarizide und anthelmintische Zusammensetzungen, die eine Verbindung der Formel (I) nach einem der Ansprüche 1 bis 5, zusammen mit einem inerten Verdünnungsmittel oder Träger, umfaßt.

7. Zusammensetzung nach Anspruch 6 in Form eines flüssigen Trankes oder einer oralen, Pour-on-Spot-on-Formulierung oder in Form eines Tierfutters oder eines Prämixes oder einer Ergänzung zur Zugabe zu einem Tierfutter.

8. Verfahren zum Bekämpfen von landwirtschaftlichem oder gartenbaulichem Schädlingsbefall, das Applizieren einer wirksamen Menge einer Verbindung der Formel (I) nach einem der Ansprüche 1 bis 5 auf den Organismus, der für die Infektion oder für den Befall ursächlich ist, oder den Ort davon umfaßt.

9. Verwendung einer Verbindung nach Anspruch 1 zur Herstellung einer Zusammensetzung zur Behandlung von Insekten- oder Parasiteninfektionen oder -Befall bei Menschen oder Tieren.

## Revendications

1. Composé de formule : 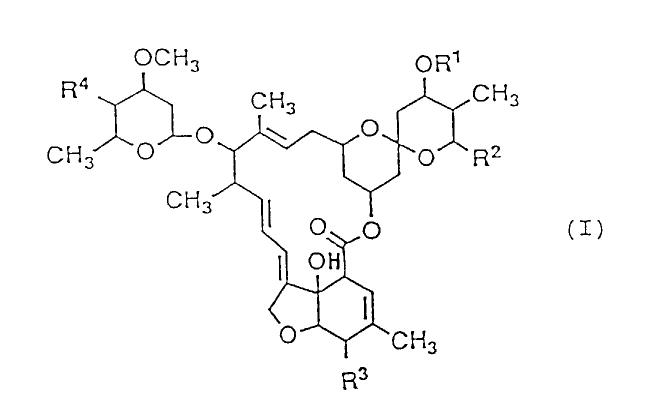 dans laquelle :
R¹ représente alkyle en C₁-C₆ ;
R² représente cycloalkyle en C₃-C₈ ;
R³ représente OH, alcoxy en C₁-C₄ ou R³ est lié par une double liaison et représente =N-OH ; et
R⁴ représente HO ou un groupe de formule :

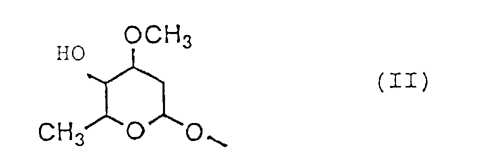

2. Composé selon la revendication 1, dans lequel R³ représente OH.

3. Composé selon la revendication 1, dans lequel R³ représente =N-OH.

4. Composé selon la revendication 1, dans lequel R¹ représente méthyle ou éthyle.

5. Composé selon l'une quelconque des revendications 1 à 4, dans lequel R² représente cyclohexyle.

6. Composition pour le traitement et la prévention d'infections parasitaires chez l'homme et l'animal, comprenant des compositions ectoparasiticides, insecticides, acaricides et anthelmintiques, qui comprend un composé de formule (I) selon l'une quelconque des revendications 1 à 5 avec un diluant ou un support inerte.

7. Composition selon la revendication 6, sous la forme d'un breuvage liquide ou d'une formulation orale ou à verser ou à tamponner, ou sous la forme d'un aliment pour animaux ou d'un prémélange ou d'un supplément à ajouter à l'alimentation animale.

8. Procédé pour combattre des infestations par des insectes nuisibles à l'agriculture ou à l'horticulture, qui consiste à appliquer une quantité efficace d'un composé de formule (I) selon l'une quelconque des revendications 1 à 5 à l'organisme responsable de ladite infection ou infestation ou au lieu infecté ou infesté.

9. Utilisation d'un composé de formule (I) pour la préparation d'une composition pour traiter des infections ou des infestations par insecte ou parasite chez l'homme ou l'animal.
